# FASCICULE DE BREVET EUROPEEN

(11) **EP 3 691 604 B1**
(45) Date de publication et mention de la délivrance du brevet: **13.09.2023**
(21) Numéro de dépôt: 18793253.8
(22) Date de dépôt: 05.10.2018
(51) Int. Cl.: A61K 8/19, A61K 8/25, A61Q 1/06, A61K 8/73

(54) **COMPOSITION DE ROUGE A LEVRES D'ASPECT MAT**
MATTE LIPPENSTIFTZUSAMMENSETZUNG
MATTE LIPSTICK COMPOSITION

(30) Priorité: 05.10.2017 FR 1759358
(43) Date de publication de la demande: 12.08.2020
(73) Titulaire: Chanel Parfums Beauté, 92200 Neuilly-sur-Seine (FR)
(72) Inventeur: FIOLEAU, Hélène, 75015 Paris (FR); DE CLERMONT-GALLERANDE, Hélène, 94300 Vincennes (FR); MALVEZIN, Chantal, 60260 Lamorlaye (FR)
(74) Mandataire: Plasseraud IP
(86) Numéro de dépôt international: PCT/FR2018/052458
(87) Numéro de publication internationale: WO 2019/069035

(56) Documents cités:
- JP-A- 2005 220 053
- JP-A- 2010 083 792
- JP-A- 2012 176 910
- JP-B2- 2 893 541
- US-A- 5 672 339
- US-A1- 2015 313 812

## Description

### Domaine technique de l'invention

L'invention concerne le domaine des produits cosmétiques, et plus particulièrement les compositions cosmétiques solides d'aspect mat et intense en couleur tels que des bâtons de rouge à lèvres mats et intenses.

### Arrière-plan technique

L'utilisation des bâtons à lèvres comme moyen de coloration des lèvres a subi une croissance rapide, de sorte que les rouges à lèvres sont aujourd'hui des produits de beauté largement répandus. Un rouge à lèvres cosmétiquement acceptable doit s'étaler facilement, être de couleur homogène et présenter un point de fusion supérieur à la température du corps. Par ailleurs, il doit donner aux lèvres un aspect lisse mais non gras et doit garder sa consistance sans que des phénomènes d'exsudation, de suintement, de rupture ou de désagrégation ne se produisent.

Un rouge à lèvres doit non seulement présenter les qualités mentionnées ci-dessus, mais surtout impartir aux lèvres une sensation lisse et crémeuse et les protéger d'un dessèchement ou de gerçures. Il est notamment difficile d'obtenir une telle sensation lisse et crémeuse à l'aide des rouges à lèvres ayant un lustre ou brillant réduit, à savoir un aspect mat.

De manière courante, les rouges à lèvres mats contiennent diverses argiles et silices en vue d'obtenir un aspect mat. L'emploi d'argiles et de silices conduit à un bâton dur qui devient toujours plus sec et dur lorsque la teneur en substances solides augmente. Par ailleurs, lors de l'application sur les lèvres d'un tel rouge à lèvres mat on obtient une sensation de sécheresse. Il en résulte donc un certain besoin de réaliser un rouge à lèvres qui assure une application crémeuse tout en permettant un aspect mat, ce qui jusqu'à présent était indissociable d'une texture sèche.

La demande de brevet JP2005/220053 décrit des compositions cosmétiques sous forme de poudre et comprenant du carbonate de magnésium tubulaire. La demande de brevet US5672339 décrit des compositions de rouge à lèvres comprenant des particules de silice ayant une surface spécifique de 10 m²/g. La demande de brevet US2015/313812 décrit des compositions mattes, non desséchantes et confortables.

La présente invention est définie par les revendications.

La présente invention a pour premier objet de fournir une composition de rouge à lèvres ayant un aspect mat et intense en couleur et une texture homogène et légère et qui le reste au cours du temps.

La présente invention est caractérisée par le fait que la composition de rouge à lèvres mat comprend au moins un agent structurant, au moins une huile, au moins un agent colorant, et au moins une charge particulière en tant qu'agent matifiant.

Ainsi, l'invention a pour objet une composition de rouge à lèvres d'aspect mat comprenant au moins un agent structurant, au moins une huile, au moins un agent colorant et au moins une charge particulière comme agent matifiant, caractérisée en ce que la charge particulière comme agent matifiant est choisie parmi les poudres de carbonate de magnésium, en particulier le carbonate de magnésium basique de formule (MgCO₃)₃.Mg(OH)₂.3H₂O ou de formule (MgCO₃)₄.Mg(OH)₂.5H₂O ou bien le carbonate de magnésium normal de formule (MgCO₃).xH₂O, parmi les terres de diatomées, et parmi la cellulose microcrystalline, ou un de leurs mélanges, les particules ayant une surface spécifique inférieure à 30 m²/g, de préférence inférieure à 20 m²/g, de façon encore plus préférée inférieure à 10m²/g et de façon encore plus avantageuse inférieure à 5 m²/g.

Dans un mode de réalisation particulier selon l'invention, la composition de rouge à lèvres d'aspect mat comprend au moins un agent structurant, au moins une huile, au moins un agent colorant et au moins une charge particulière comme agent matifiant, caractérisée en ce que la charge particulière comme agent matifiant est choisie parmi les poudres de carbonate de magnésium ayant une surface spécifique inférieure à 10m²/g et de façon encore plus avantageuse inférieure à 5 m²/g.

### La charge particulière utilisée en tant qu'agent matifiant

Par charge on entend des particules solides destinées à être dispersées dans le milieu de la composition et qui restent insolubles dans ce milieu, quelle que soit la température à laquelle la composition est fabriquée et stockée.

La charge particulière utilisée en tant qu'agent matifiant de l'invention est une particule caractérisée par sa taille, sa forme et sa surface spécifique.

La taille de la charge particulière utilisée mesurée en prenant les extrémités les plus éloignées de la particule est comprise entre 1 micron (µm) et 30 microns (µm). Plus sa taille est petite plus son fini est mat.

La forme de la charge particulière utilisée est lamellaire ou cubique ou sphérique ou arrondie si elle est d'une taille inférieure à 10 microns. En revanche lorsque sa taille est supérieure à 10 microns sa forme est de préférence sphérique ou arrondie pour éviter la réflexion spéculaire et donc un effet satiné ou brillant.

La surface spécifique de la charge particulière utilisée est inférieure à 30 m²/g, de préférence la surface spécifique est inférieure à 20 m²/g, de façon encore plus préférée la surface spécifique est inférieure à 10 m²/g et de façon encore plus avantageuse la surface spécifique est inférieure à 5 m²/g.

Les mesures de surface spécifique des charges matifiantes peuvent être réalisées à l'aide d'un appareil de mesure BET tristar II. La méthode est basée sur l'adsorption des molécules d'azote à la surface et dans les pores de l'échantillon de poudre à basse température (température de liquéfaction de l'azote : 77°K). La surface spécifique est exprimée en m²/g.

Parmi les charges particulières utilisables dans la présente invention, on peut citer le carbonate de magnésium. Plus spécifiquement la charge particulière est choisie parmi les poudres de carbonate de magnésium, en particulier le carbonate de magnésium basique de formule (MgCO₃)₃.Mg(OH)₂.3H₂O ou de formule (MgCO₃)₄.Mg(OH)₂.5H₂O ou bien le carbonate de magnésium normal de formule (MgCO₃).xH₂O. On peut citer, à titre d'exemple, le carbonate de magnésium commercialisé par la société ICL Industrial products sous l'appelation de "basic magnésium carbonate". Les particules sont présentes à des tailles comprises entre 5 microns et 25 microns, elles ont une forme de roses des sables et leur surface spécifique est d'environ 4 m²/g.

Parmi les charges particulières utilisables dans la présente invention, on peut citer également les terres de diatomée. On peut citer en particulier les terres de diatomées commercialisées par la société Imerys sous l'appellation de diatomée Imercare^{®} 03D. Les particules sont présentes à des tailles comprises entre 2 microns et 5 microns, elles ont des formes diverses et leur surface spécifique est d'environ 12 m²/g.

On peut citer également les terres de diatomées commercialisées par la société Imerys sous l'appellation de "diatomée Imercare^{®} 400D. Les particules sont présentes à des tailles comprises entre 15 microns et 25 microns, elles ont des formes diverses et leur surface spécifique est d'environ 19 m²/g.

Parmi les charges particulières on peut citer la cellulose microcrystalline. On peut citer notamment celle commercialisée par Rettenmaier sous l'appellation Vivapur^{®} CS 4FM. Les particules sont présentes à des tailles comprises entre 0.9 microns et 9.4 microns et leur surface spécifique est d'environ 5.7 m²/g.

Parmi les charges particulières on peut citer également une poudre de bambou. On peut citer notamment celle commercialisée par Rossow sous l'appellation Sencocel^{®} BC20.

On préfère les charges particulières choisies parmi le carbonate de magnésium basique, les terres de diatomées, la cellulose microcristalline, ou un de leurs mélanges.

La charge particulière utilisée comme agent matifiant peut être utilisée telle quelle ou bien peut être traitée en surface par un agent hydrophobe.

Parmi les agents hydrophobes on peut citer notamment les silanes, silicones, savons d'acides gras, C₉-₁₅ fluoroalcool phosphates, copolymères acrylate/dimethicone, copolymères mixtes C₉-₁₅ fluoroalcool phosphates / silicones, lécithines, cire de carnauba, polyéthylène, chitosan et acides aminés éventuellement acylés tels que la lauroyl lysine, le disodium stearoyl glutamate et l'aluminium acyl glutamate.

Selon l'invention, la charge particulière utilisée comme agent matifiant est présente en une proportion comprise entre 8% et 20% et de préférence entre 10% et 15% en poids par rapport au poids total de la composition.

### L'huile

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Dans le contexte de la présente invention, le terme "huile" comprend également des graisses lipophiles qui sont capables de subir un changement liquide/solide réversible et ont une organisation cristalline anisotrope à l'état solide, mais qui sont différents des cires par le fait qu'elles contiennent, à une température de 23°C, une fraction liquide et une fraction solide. Un composé de ce type est notamment un mélange d'esters de stérols, tel que le mélange de cholestérol et d'ester de lanosterol disponibles auprès du fabricant CRODA sous la dénomination commerciale Super Sterol Ester^{®}.

### Huile non volatile

On entend par « huile non volatile » une huile qui présente une température d'ébullition généralement supérieure à 300°C sous 760 mm de Hg (101325 Pa) et qui ne présente pas ou peu de tension de vapeur.

Les huiles non volatiles peuvent notamment être choisies parmi les huiles de silicone non volatiles, les huiles hydrocarbonées non volatiles, et leurs mélanges.

On entend par « huile de silicone » une huile comprenant au moins un atome de silicium, et notamment au moins un groupe Si-O.

Comme huile de silicone non volatile, on peut notamment citer les polydiméthylsiloxanes renfermant au moins 8 atomes de silicium, les polyalkylméthylsiloxane dont la chaîne alkyle renferme de 8 à 20 atomes de carbone et les huiles identifiées par le nom INCI phenyl trimethicone.

On entend par "huile hydrocarbonée", une huile contenant uniquement des atomes d'hydrogène et de carbone.

On peut citer par exemple les hydrocarbures tels que le squalane, le phytosqualane, le polybutène, le polyisobutène hydrogéné, le polydécène hydrogéné, les (poly) esters synthétiques encore appelées "huiles ester" et (poly) éthers, en particulier les (poly) esters d'acides en C6-C20 et d'alcools en C6-C20, avantageusement ramifiés tels que l'isononanoate d'isononyle ; les huiles végétales ; les acides gras ramifiés et/ou insaturés ; les alcools gras ramifiés et/ou insaturés tels que l'octyldodécanol ; ou un de leurs mélanges.

On entend par « huile ester », une huile mono-, di-, tri- ou tétra-ester. Les huiles esters sont obtenues en faisant réagir un mono-, di-, tri- et plus généralement un polyol avec un mono- di- tri- et plus généralement un poly-acide carboxylique, lesdits réactifs pouvant être linéaires ou ramifiés, saturés ou insaturés, aliphatiques ou aromatiques, et pouvant éventuellement comprendre des groups alcoxylés. Les huiles esters peuvent notamment être hydroxylées.

En particulier, l'huile ester non volatile peut comprendre de 18 à 70 atomes de carbones.

L'huile ester non volatile peut notamment être choisie parmi :
- les monoesters comprenant 18 à 40 atomes de carbone, en particulier les monoesters de formule R1COOR2 dans laquelle R1 représente le reste d'un acide gras linéaire ou ramifié comportant de 6 à 20 atomes de carbone et R2 représente une chaîne hydrocarbonée notamment ramifiée contenant de 6 à 20 atomes de carbone, comme par exemple l'huile de Purcellin (octanoate de cétostéaryle), l'isononanoate d'isononyle, le néopentanoate d'isodécyle, les benzoates d'alkyles en C12 à C15, le palmitate de 2-éthylhexyle, le néopentanoate d'octyledodécyle, le stéarate de 2-octyldodécyle, l'érucate de 2-octyldodécyle, l'isostéarate d'isostéaryle, le benzoate de 2-octyldodécyle, des octanoates, décanoates ou ricinoléates d'alkyles, le myristate d'isopropyle, le palmitate d'isopropyle, le stéarate de butyle, le laurate d'hexyle, le palmitate de 2-éthylhexyle, le laurate de 2-hexyldécyle, le palmitate de 2-octyldécyle, le myristate de 2-octyldodécyle, le succinate de 2-diéthylhexyle ;
- les diesters comprenant 18 à 60 atomes de carbone, en particulier de 18 à 50 atomes de carbone, comme les diesters de diacide carboxylique et de monoalcools, tel que le malate de diisostéaryle ; les diesters de glycol et de monoacides carboxyliques, tels que le diheptanoate de néopentylglycol ou le polyglycéryle-2 diisostéarate ;
- les triesters comprenant 35 à 70 atomes de carbone, comme les triesters de triacide carboxylique, tels que le triisostéaryle citrate ou le tridécyl trimellitate ; ou les triesters de glycol et de monoacides carboxyliques tels que le polyglycéryl-2 triisostéarate;
- les tétraesters comprenant 35 à 70 atomes de carbone, tels que les tétraesters de penthaérythritol ou de polyglycérol et d'un monoacide carboxylique, par exemple le tétrapélargonate de pentaérythrityle, le tétraisostéarate de pentaérythrityle, le tétraisononanoate de pentaérythrityle, le tridécyl-2 tétradécanoate de glycéryle, le tétraisostéarate de polyglycéryle-2 ou encore le tétradécyl-2 tétradécanoate de pentaérythrityle;
- les polyesters obtenus par condensation de dimère et/ou trimère d'acide gras insaturé et de diol tels que ceux décrits dans la demande de brevet FR 0 853 634, comme le polyester de l'acide dilinoléique et du 1,4-butanediol ;
- les esters et polyesters de dimère diol et d'acide mono- ou dicarboxylique, tels que les esters de dimère diol et d'acide gras et les esters de dimère diols et de dimère diacide carboxylique, en particulier ceux obtenus à partir d'un dimère d'un acide gras insaturé en C8 à C34, notamment en C12 à C22, en particulier en C16 à C20, et plus particulièrement en C18, tels que les esters de diacides dilinoléiques et de dimères diols dilinoléiques, par exemple ceux commercialisés par la société NIPPON FINE CHEMICAL sous la dénomination commerciale LUSPLAN DD-DA5^{®} et DD-DA7^{®} ;
- les triglycérides d'acides gras (liquides à température ambiante), notamment d'acides gras ayant de 7 à 40 atomes de carbone, tels que les triglycérides des acides heptanoïque ou octanoïque ou l'huile de jojoba ; les triglycérides saturés tels que le caprylic/capric triglycéride, le triheptanoate de glycéryle, le trioctanoate de glycérine ; les triglycérides d'acide en C18-36 tels que ceux commercialisés sous la référence DUB TGI 24 commercialisé par Stéarineries Dubois) ; et les triglycérides insaturés tels que l'huile de ricin, l'huile d'olive, l'huile de ximénia, l'huile de pracaxi ;
- ou un de leurs mélanges.

L'huile non volatile utilisée dans la présente invention est de préférence une huile faiblement brillante.

On appelle une huile faiblement brillante une huile dont l'indice de réfraction est inférieur à 1,46 de préférence inférieur à 1,45, encore plus préférée inférieur à 1,44.

L'indice de réfraction est mesuré à l'aide d'un réfractomètre d'ABBE paralux ref 60-6400-9.

Parmi les huiles faiblement brillantes utilisables dans l'invention on peut citer par exemple les (poly) esters et (poly) éthers synthétiques, en particulier les (poly) esters d'acides en C6-C20 et d'alcools en C6-C20, avantageusement ramifiés, tels que l'isononanoate d'isononyle; les di(alkyl en C6-C20) carbonates tels que le dicaprylyl carbonate commercialisé par BASF sous la dénomination Cetiol CC ; les acides gras ramifiés et/ou insaturés ; les polyesters de polyols, en particulier de (di) pentaérythritol, tels que le tétraoctanoate de pentaérythritol, les huiles de silicone telles que les polydiméthylsiloxanes linéaires de viscosité comprise entre 5×10⁻⁶m²/s et 10⁻⁴m²/s ; ou un de leurs mélanges.

Parmi les huiles faiblement brillantes utilisables dans l'invention on utilise de préférence les huiles de faible viscosité. Par faible viscosité on entend une viscosité inférieure à 1000 Pa.s, de préférence inférieur à 500 Pa.s, encore plus préférée inférieur à 100 Pa.s et de façon encore plus avantageuse inférieur à 50 Pa.s.

La viscosité est mesurée à l'aide d'un rhéomètre Gémini vendu par la société Malvern Instruments. Une mesure est réalisée en rotation en balayage contrainte de 0.01Pa à 1000Pa. On utilise un système couette avec cylindres coaxiaux de 25mm de diamètre (C25) avec un entrefer de 150 micron à une température de 20°C. La valeur indiquée pour chaque huile est la moyenne obtenue sur plusieurs points du plateau.

Parmi ces huiles faiblement brillantes et de faible viscosité on peut citer l'isononyl isononanoate, le dicaprylyl carbonate ou un de leurs mélanges.

Cela permet en effet d'incorporer davantage de charge particulière comme agent matifiant et davantage de pigments et donc d'obtenir une couleur à la fois matte et intense.

On peut utiliser également une huile additionnelle non volatile pour apporter des propriétés supplémentaires à la composition de l'invention.

A titre d'exemple on peut ajouter du diisostearyl malate car il permet d'obtenir une bonne dispersion des pigments.

On peut ajouter d'autres huiles additionnelles pour améliorer les propriétés sensorielles de la formule.

Selon un mode de réalisation, l'huile non volatile est choisie parmi l'isononyl isononanoate, le dicaprylyl carbonate, le diisostéaryl malate ou un de leurs mélanges.

Selon un mode de réalisation particulier de l'invention, l'huile introduite dans la composition selon l'invention est un mélange d'isononyl isononanoate, de dicaprylyl carbonate et de diisostéaryl malate.

L'huile peut être présente dans la composition selon l'invention en une teneur comprise entre 40% et 80% en poids, de préférence entre 45% et 70% en poids, plus préférentiellement entre 50% et 60% en poids, par rapport au poids de la composition.

### Huile volatile

Selon un mode de réalisation avantageux de l'invention, la composition ne contient pas (0%) ou alors très peu (maximum 5% en poids par rapport au poids total de la composition) d'huile volatile.

Par "huile volatile", on entend une huile susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle à température ambiante et pression atmosphérique, en particulier ayant une pression de vapeur comprise entre 0,13 Pa et 40 000 Pa (0,001 à 300 mm de Hg), de préférence comprise entre 1,3 Pa et 13 000 Pa (0,01 à 100 mm de Hg), et plus préférentiellement encore comprise entre 1,3 Pa et 1 300 Pa (0,01 à 1000 mm de Hg).

Les huiles volatiles comprennent les huiles siliconées volatiles et/ou les huiles hydrocarbonées volatiles.

Les huiles siliconées volatiles éventuellement utilisées dans les compositions de l'invention sont linéaires ou cycliques, ont notamment de 2 à 7 atomes de silicium, éventuellement des groupes alkyle ou alkoxy ayant de 1 à 10 atomes de carbone, et présentent une viscosité, à température ambiante, inférieure à 5 cSt.

A titre d'exemples d'huile siliconée volatile on pourra plus particulièrement citer l'hexaméthylcyclotrisiloxane, l'octaméthylcyclotétrasiloxane, le décaméthylcyclopentasiloxane, le dodecaméthylcyclohexasiloxane, le cyclotétradiméthylsiloxane, le cyclopentadiméthylsiloxane, le cyclohexadiméthylsiloxane, l'hexaméthyldisiloxane, l'octaméthyltrisiloxane, l'hexylheptaméthyltrisiloxane, l'octylheptaméthyltrisiloxane, le décaméthyltétrasiloxane, le dodécaméthylpentasiloxane, l'heptaméthylhexyl trisiloxane, l'heptaméthyloctyl trisiloxane ou un de leurs mélanges.

Concernant l'huile hydrocarbonée volatile on pourra plus particulièrement citer une huile hydrocarbonée à chaîne courte, les alcanes linéaires volatiles tels que par exemple décrits dans le document FR2933865 incorporé par référence.

A titre d'exemples d'huile(s) hydrocarbonée(s) à chaîne courte on pourra notamment citer celle(s) choisie(s) dans le groupe comprenant l'isododécane, l'isodécane, l'isohexadécane, le dodécane ou un de leurs mélanges.

A titre d'exemple d'alcanes linéaires volatiles, on pourra citer ceux ayant des chaînes hydrocarbonées en :
- C9-C17, C10-C14, tel qu'un mélange de undécane et tridécane, commercialisé par BASF Care Créations sous la dénomination Cetiol^{®} Ultimate,
- C15-19, tel que ceux commercialisés par Seppic sous la dénomination Emogreen L15,
- C12-14, tel que ceux commercialisés par Biosynthis sous la dénomination Vegelight 1214LC.
- C 9-12 alkane, tel que ceux commercialisés parDaito sous la dénomination Makigreen D10.

### L'agent structurant

L'agent structurant de la phase huileuse comprend au moins une cire et/ou au moins une résine siliconée et/ou un gélifiant lipophile.

Le terme «cire» désigne une matière grasse à changement réversible liquide/solide, ayant une température de fusion supérieure à 30°C et généralement inférieure à 110°C, qui est liquide dans les conditions de préparation de la composition et qui présente une organisation cristalline anisotrope à l'état solide.

Le terme « gélifiant lipophile», désigne une substance capable de solidifier ou de gélatiniser l'huile introduite dans la composition de l'invention.

Selon un mode de réalisation de l'invention, l'agent structurant est un mélange d'au moins une cire et d'au moins un gélifiant lipophile.

Selon un autre mode de réalisation de l'invention, l'agent structurant est un mélange d'au moins une cire et d'au moins une résine siliconée.

Selon un autre mode de réalisation de l'invention, l'agent structurant est un mélange d'au moins deux cires.

La cire appropriée pour les compositions cosmétiques de l'invention comprend au moins une cire polaire et/ou au moins une cire apolaire.

Par cire polaire, on entend une cire comprenant au moins un hétéroatome tel que l'oxygène, l'azote, le silicium ou le phosphore.

En particulier, la cire polaire peut être choisie dans le groupe comprenant la cire d'abeille, la cire de carnauba, la cire de candelilla, la cire de coton, la cire de son de riz, la cire de baie, la cire d'insecte de chine, la cire de montan, la lanoline et ses dérivés alcools, acétylés, estérifiés, polyéthoxylés, la cire de kapok, la cire de canne à sucre, le laurate d'hexyle, la cire de jojoba, la cire shellac, l'éther de cholestérol polyéthoxylé, les cires d'abeille synthétiques commercialisées par Koster Keunen sous la dénomination commerciale Kester Wax K82H, ou un de leurs mélanges.

On peut également citer les cires d'esters végétaux choisies dans le groupe comprenant le mélange d'esters de jojoba, de polyglycérine-3, de cire de fleur d'*Acacia decurrens* et de cire de graines de tournesol, ledit mélange étant commercialisé par Gattefosse sous la dénomination commerciale Acticire^{®}, les esters de jojoba commercialisés par Floratech sous la dénomination commerciale Floraesters 60 ou Floraesters 70, les esters d'alkyle ou d'esters d'alkyle hydrogénés commercialisés par Sophim sous le nom commercial Phytowax, comme par exemple les esters d'oléate de lauroyl hydrogéné commercialisés sous la dénomination Phytowax Olive 12L44.

Par cire apolaire, on entend une cire hydrocarbonée et/ou une cire siliconée.

On entend par « cire apolaire hydrocarbonée », une cire comprenant uniquement des atomes de carbone et d'hydrogène et ne comprenant pas d'hétéroatomes tels que l'oxygène, l'azote, le silicium ou le phosphore.

Des exemples de cires apolaires hydrocarbonées appropriées dans les compositions de l'invention comprennent la cire de polyéthylène, commercialisée par New Phase Technologies sous la dénomination Performalène 400 (P400) ou par Jeen International Corporation sous la dénomination Jeenate 3H, un mélange de polyéthylène linéaire de haut poids moléculaire et de copolymère éthylène/propylène, commercialisé par Safic-Alcan sous la dénomination Lipwax^{®} PZ80-20, une cire synthétique commercialisée par Sasol sous la dénomination Sasol Wax C80, des cires synthétiques et des cires végétales, comme par exemple un mélange de cire synthétique et de cire de carnauba (Copernica cerifera) commercialisé par Strahl & Pitsch sous la dénomination Smart wax 202, un mélange de cire synthétique, de cire Candelilla et de cire de carnauba (Copernica cerifera) commercialisé par Strahl & Pitsch sous la dénomination Smartwax 7743S, les cires de Fischer Tropsch commercialisées par Cirebelle sous la dénomination Cirebelle 303, ou un de leurs mélanges.

On entend par « cire apolaire siliconée », une cire comprenant un hétéroatome de silicium.

Des exemples de cires apolaires siliconées appropriées dans les compositions de l'invention comprennent la C20-24 alkyl diméthicone, commercialisée par Siltech sous la dénomination Silwax D2024, la C24-28 alkyle diméthicone, commercialisée par Evonik Industries AG sous la dénomination Abil Wax, ou un de leurs mélanges.

Plus particulièrement, selon encore un autre mode de réalisation de l'invention, la cire est choisie dans le groupe comprenant la cire d'abeille, la cire de polyéthylène, commercialisée par New Phase Technologies sous la dénomination Performalène 400 (P400) ou par Jeen International Corporation sous la dénomination Jeenate 3H, un mélange de polyéthylène linéaire de haut poids moléculaire et de copolymère éthylène/propylène, commercialisé par Safic-Alcan sous la dénomination Lipwax^{®} PZ80-20, une cire synthétique commercialisée par Sasol sous la dénomination Sasol Wax C80, un mélange de polyéthylène linéaire de haut poids moléculaire et de copolymère éthylène/propylène, commercialisé par Safic-Alcan sous la dénomination Lipwax^{®} PZ80-20.

Ainsi selon un mode préféré de l'invention, l'agent structurant est une cire choisie parmi la cire d'abeille, la cire synthétique, la cire de polyéthylène ou un de leurs mélanges.

Selon un mode de réalisation de l'invention, la cire est présente dans la composition cosmétique de l'invention en une teneur comprise entre 10% et 30%, de préférence entre 15% et 25%, les pourcentages étant des pourcentages en poids par rapport au poids total de la composition.

A titre d'exemple des résines siliconées, on peut citer :
- les siloxysilicates qui peuvent être des triméthylsiloxysilicate de formule [(CH3)3SiO]x(SiO4/2)y (unités MQ) dans laquelle x et y sont des entiers compris entre 50 et 80,
- les polysilesquioxanes de formule (CH3SiO3/2).x (unités T) dans laquelle x est supérieur à 100 et dont au moins un des radicaux méthyle peut être substitué par un groupement R tel que défini plus haut, les polyméthylsilsesquioxanes qui sont des polysilsesquioxanes dans lesquels aucun des radicaux méthyle n'est substitué par un autre groupement. De tels polyméthylsilsesquioxanes sont décrits dans le document US 5,246,694.

A titre d'exemples de résines polyméthylsilsesquioxanes commercialement disponibles, on peut citer celles qui sont commercialisés :
- par la société Wacker sous la référence Resin MK tels que la Belsil PMS MK : polymère comprenant des unités répétitives CH3SiO3/2 (unités T), pouvant aussi comprendre jusqu'à 1% en poids d'unités (CH3)2SiO2/2 (unités D) et présentant un poids moléculaire moyen d'environ 10000,
- par la société SHIN-ETSU sous les références KR-220L qui sont composés d'unités T de formule CH3SiO3/2 et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88% d'unités T et 12% d'unités dimethyl D et ont des groupes terminaux Si-OH.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.

Les gélifiants lipophiles pouvant être utilisés dans les compositions selon l'invention peuvent être des gélifiants lipophiles organiques ou minéraux, polymériques ou moléculaires.

Comme gélifiant lipophile minéral, on peut citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées " Silica silylate " selon le CTFA (8ème édition, 2000). Elles sont par exemple commercialisées sous les références Aerosil R812^{®} par la société DEGUSSA, CAB-O-SIL TS-530^{®} par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées " Silica diméthyl silylate " selon le CTFA (8ème édition, 2000 ). Elles sont par exemple commercialisées sous les références Aerosil R972^{®}, et Aerosil R974^{®} par la société DEGUSSA, CAB-O-SIL TS-610^{®} et CAB-O-SIL TS-720^{®} par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C1 à C6, et en particulier en C1 à C3 ou un de leurs mélanges. Les copolymères séquencés de type " dibloc ", " tribloc " ou " radial " du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960) ou dans le polyisobutène hydrogéné (Versagel ME 2000).

Un autre type de gélifiant lipophile organique polymérique est constitué des résines de polyamide ou de résines de poly (ester-amide), telles que les polyamides à terminaison ester (ETPA), les poly (ester-amides) terminés par un ester (ETPEA), les polyamides à terminaison amide tertiaire (ATPA), les polyamides à terminaison polyalkylèneoxy (PAOPA) ou les polyéther polyamides (PEPA).

Des exemples de polyamides à terminaison ester (ETPA) sont ceux identifiés par le nom INCI "Ethylenediamine / Stearyl Dimer Dilinoleate Copolymer" et disponibles, par exemple, sous le nom commercial Uniclear^{®} 100VG de la société Arizona Chemical.

Des exemples de poly (ester-amides) terminés par un ester (ETPEA) sont ceux identifiés par le nom INCI polyamide-8 qui sont des "Copolymères dimère dibenzoate d'éthylènediamine bis-stéaryl éthylènediamine / néopentylglycol / stéaryle" et disponibles, par exemple, sous la dénomination commerciale Oloecraft^{®} LP-20- PA-MV de la société Croda.

Des exemples de polyamides à terminaison amide tertiaire (ATPA) sont ceux identifiés par le nom INCI "Ethylènediamine / Copolymère de diilinoate de dimère hydrogéné Bis-Di-C14-18 Alkyl Amide" et disponibles, par exemple, sous la dénomination commerciale Sylvaclear^{®} A200V ou Sylvaclear^{®} A2614V de la société Arizona Chemical ou ceux identifiés par le nom INCI "malate de diisostéaryle et bis-dioctadécylamide dimère acide dilinoleique / éthylènediamine" et disponibles, par exemple, sous la dénomination commerciale Haimalate PAM de la société Kokyu Alcohol Kogyo.

Des exemples de polyamides à terminaison polyalkylèneoxy (PAOPA) sont ceux identifiés par le nom INCI Polyamide-3 et disponibles, par exemple, sous la dénomination Sylvaclear^{®} AF1900V, Sylvaclear^{®} PE1800V et Sylvaclear^{®} PA1200V de la société Arizona Chemical.

Des exemples de polyéther polyamides (PEPA) sont ceux identifiés par le nom INCI Polyamide-6 et disponibles, par exemple, sous la dénomination Sylvaclear^{®} PE400V de la société Arizona Chemical.

Un autre type de gélifiant lipophile organique polymérique est constitué des diamides d'acide N-acyl glutamique. On pourra notamment citer un diamide d'acide N-acyl glutamique ayant un groupe alkyle à chaîne droite tel que le dibutyl lauroyl glutamide et un diamide d'acide N-acyl glutamique ayant un groupe alkyle à chaîne ramifiée, tels que le dibutyl éthylhexanoyl glutamide. Le dibutyl lauroyl glutamide est disponible dans le commerce en tant que GP-1 et le dibutyl éthylhexanoyl glutamide est disponible dans le commerce sous le nom EB-21, et sont tous deux commercialisés par Ajinomoto.

Un autre type de gélifiant lipophile organique polymérique est constitué des esters de dextrine. On pourra citer les esters de dextrine et d'acides gras, tels que le palmitate de dextrine.

Un autre type de gélifiant lipophile organique polymérique est constitué des esters de saccharose. On pourra citer les esters de saccharose et d'acides gras, tels que le triacétate de tétrastéarate de saccharose disponible sous la dénomination commerciale Sisterna^{®} A10E-C de la société Sisterna.

Un autre type de gélifiant lipophile organique polymérique est constitué des esters de glycéryle. On pourra citer le diester d'acide eicosadioïque et de glycérol estérifié par l'acide béhénique. Il est en particulier disponible sous la dénomination commerciale NOMCORT^{®} HK-G de la société NISSHIN OILLIO.

Avantageusement, la composition selon l'invention peut comprendre de 0,5 à 40% en poids d'agents structurants autres que les cires par rapport au poids total de la composition, de préférence de 1 à 30% ou encore mieux, de 5 à 30%.

### Composés pâteux

La composition selon l'invention peut comprendre en outre un composé pâteux qui peut être avantageusement choisi parmi :
- la lanoline et ses dérivés
- les composés siliconés polymères ou non
- les composés fluorés polymères ou non
- les polymères vinyliques, notamment :
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C8-C30
   - les oligomères homo et copolymères d'esters vinyliques ayant des groupements alkyles en C8-C30
   - les oligomères homo et copolymères de vinyléthers ayant des groupements alkyles en C8-C30,
- les polyéthers liposolubles résultant de la polyéthérification entre un ou plusieurs diols en C2-C100, de préférence en C2-C50,
- les mélanges de cire d'abeille et d'octyldodécanol tel que celui commercialisé sous la dénomination Zenibee Cream par la société Zenitech,
- les esters,
- les beurres végétaux tels que beurre de mangue, de karité, de cacao, de coton, d'avocat...
ou un de leurs mélanges.

Parmi les esters, on peut utiliser notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol tel que le produit répondant au nom INCI « Phytosteryl/behenyl/octyldodecyl/isostearyl lauroyl glutamate » commercialisé sous la dénomination de Eldew-PS308 par la société Ajinomoto,
- les triglycérides d'acides gras et leurs dérivés, par exemple le mélange de stéaryl heptanoate et de stéaryl caprylate commercialisé sous la dénomination DUB solide par la société Stéarinerie Dubois,
- les esters de pentaérythritol,
- les polyesters non réticulés résultant de la polycondensation entre un acide dicarboxylique ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique tel que le cetyl lactate commercialisé sous la dénomination céraphyl 28 par la société ISP (International Speciality Products),
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ^{®}, et Risocast DA-L ^{®},
ou un de leurs mélanges.

Parmi les composés pâteux, on choisira de préférence :
- les composés siliconés polymères ou non
ou un mélange de ceux-ci.

La composition selon l'invention peut comprendre une teneur totale en composés pâteux comprise entre 1 et 40 % en poids par rapport au poids total de la composition, en particulier entre 4% et 30 %, plus particulièrement entre 5% et 20 %.

### Charges complémentaires

La composition de rouge à lèvres peut contenir également d'autres charges.

Ces charges sont de préférence incolores ou blanches.

Les particules qui la constituent peuvent être poreuses ou non, et se présenter sous diverses formes, notamment sous forme plaquettaire, sphérique ou oblongue, quelle que soit la forme cristallographique (par exemple feuillet, cubique, hexagonale, orthorombique, etc).

Elles sont choisies de façon à ne pas perturber le résultat recherché donc à ne pas absorber les huiles et ne pas apporter de la brillance.

En particulier la charge complémentaire peut-être choisie parmi la lauroyl lysine, le nitrure de bore, les microbilles de silicone comme celles commercialisées sous l'appellation Tospearl par Toshiba par exemple, le carbonate de calcium précipité, l'hydroxyapatite, les particules de polyorganosiloxanes élastomères, les microcapsules de verre ou de céramique, le laurate de zinc, le myristate de magnésium, le silicate de magnésium et d'aluminium comme celui commercialisé sous l'appellation commerciale Neusilin ULF2 par la société Fuji Chemical Industry, de l'amidon, une argile ou un de leurs mélanges.

Parmi les charges complémentaires, on préfère l'amidon, une argile ou un de leurs mélanges.

L'amidon peut être choisi par exemple parmi un amidon de riz, de tapioca, de pomme de terre ou de maïs. On préfère l'amidon de riz en particulier celui de nom INCI distarch phosphate commercialisé sous l'appellation "Rice PO4 Naturel" par la société Agrana Starch. Il absorbe très peu les huiles et présente un aspect mat et apporte une douceur à la composition de l'invention qui évite le coté crissant de la charge particulière comme agent matifiant.

L'amidon peut-être présent dans la composition de rouge à lèvres en une quantité comprise entre 2,0% à 10,0% en poids, de préférence entre 6,0 et et 10,0% en poids par rapport au poids total de la composition.

L'argile peut être naturelle ou synthétique. Elle est rendue lipophile par un traitement avec un sel d'alkyl ammonium comme un chlorure d'ammonium en C10 à C22, par exemple le chlorure de di-stéaryl di-methyl ammonium. Elle peut être choisie parmi les bentonites en particulier les hectorites et les montmorillonites, les beidellites, les saponites, les nontronites, les sépiolites, les biotites, les attapulgites, et les vermiculites. De préférence l'argile est choisie parmi les hectorites. On peut citer à titre d'exemple d'hectorite le produit vendu sous le nom Bentone 38V CG par la société ELEMENTIS SPECIALTIES (nom INCI disteardimonium hectorite).

Elle présente l'avantage de renforcer l'effet matifiant tout en ayant un rôle de modificateur de rhéologie. Elle peut être présente en une proportion comprise entre 0,1% et 4% et de préférence comprise entre 0,5% et 2 % en poids par rapport au poids total de la composition.

### Additifs Additionnels

Mis à part les constituants précités, la composition selon l'invention peut contenir divers ingrédients, tels qu'un agent colorant, un filtre UV, ou un de leurs mélanges.

L'agent colorant peut notamment être choisi parmi les colorants hydrosolubles ou liposolubles, les pigments, les nacres, les laques ou un de leurs mélanges. Ces agents colorants peuvent être éventuellement traités en surface par un agent hydrophobe tel que les silanes, silicones, savons d'acides gras, C₉-₁₅ fluoroalcool phosphates, copolymères acrylate/dimethicone, copolymères mixtes C₉₋₁₅ fluoroalcool phosphates / silicones, lécithines, cire de carnauba, polyéthylène, chitosan et acides aminés éventuellement acylés tels que la lauroyl lysine, le disodium stearoyl glutamate et l'aluminium acyl glutamate. Les pigments peuvent être minéraux ou organiques, naturels ou de synthèse.

Des exemples de pigments minéraux sont notamment le dioxyde de titane, les oxydes de fer, de zinc ou de chrome, les violets de manganèse, les ultramarines, le ferrocyanure ferrique dit Bleu de Prusse, ainsi que les pigments composites et les pigments goniochromatiques, perlescents, interférentiels, photochromes ou thermochromes, sans que cette liste ne soit limitative.

Des exemples de pigments organiques utilisables dans l'invention sont notamment le noir de carbone, les pigments de type D&C, les laques à base de carmin de cochenille, de baryum, de strontium, de calcium ou d'aluminium ou encore les dicétopyrrolopyrrole (DPP) décrits dans les documents EP-A-542669, EP-A-787730, EP-A-787731 et WO-A-96/08537.

Les nacres peuvent être choisies parmi celles classiquement présentes dans les produits de maquillage, telles que les mica / dioxyde de titane. En variante, il peut s'agir de nacres à base de mica / silice / dioxyde de titane, à base de fluorphlogopite synthétique / dioxyde de titane (SUNSHINE^{®} de MAPRECOS), de calcium sodium borosilicate / dioxyde de titane (REFLECKS^{®} d'ENGELHARD) ou de calcium aluminium borosilicate / silice / dioxyde de titane (RONASTAR^{®} de MERCK).

Avantageusement, lorsqu'elle renferme un ou plusieurs pigments, la composition selon l'invention contient en outre au moins un dispersant tel que le diisostearyl malate.

Les agents colorants sont présents dans la composition en une teneur comprise entre 5% et 8%, les pourcentages étant des pourcentages en poids par rapport au poids total de la composition.

Les filtres UV peuvent notamment être choisis parmi les filtres organiques et inorganiques ou un de leurs mélanges. Comme filtres organiques, on peut citer notamment les dérivés de dibenzoylméthane (dont le butyl methoxydibenzoylmethane), les dérivés d'acide cinnamique (dont l'ethylhexyl methoxycinnamate), les salicylates, les acides para-aminobenzoïques, les β,β'-diphénylacrylates, les benzophénones, les dérivés de benzylidène camphre, les phénylbenzimidazoles, les triazines, les phénylbenzotriazoles et les dérivés anthraniliques. Comme filtres inorganiques, on peut notamment citer les filtres à base d'oxydes minéraux sous forme de pigments ou de nanopigments, enrobés ou non, et en particulier à base de dioxyde de titane ou d'oxyde de zinc.

La composition de l'invention peut également renfermer des beurres végétaux ou des beurres d'origine synthétique. Dans tous les cas ceux-ci sont choisis pour éviter d'apporter de la brillance. On préfère utiliser des beurres d'aspect mat tel que le beurre de mangue par exemple.

La composition selon l'invention peut également renfermer un ou plusieurs agents édulcorants tels que le sorbitol, le sucrose, le xylitol, l'acésulfame K et le saccharinate de sodium ; des anti-oxydants tels que les esters alkylés ou phosphorylés d'acide ascorbique, ou encore le tocophérol et ses esters ; des séquestrants tels que les sels d'EDTA ; des ajusteurs de pH ; des conservateurs ; des parfums ; des vitamines ; des agents hydratants ; ou un de leurs mélanges.

Des exemples de tels adjuvants sont cités notamment dans le Dictionnaire CTFA (International Cosmetic Ingrédient Dictionary and Handbook publié par The Cosmetic, Toiletry and Fragrance Association, 11ème Edition, 2006).

### Aspect mat de la composition

La composition de rouge à lèvres présente de manière avantageuse un aspect mat et une texture homogène et crémeuse.

L'aspect mat de la composition de rouge à lèvres est mis en évidence par une mesure de brillance.

La composition de rouge à lèvres de l'invention étalée sur une carte de contraste à fond blanc sur une épaisseur de 500 microns grâce à un étaleur automatique présente une valeur de brillance mesurée à 85° à l'aide d'un brillance mètre sous le nom de micro-TRI-gloss ou PICOGLOSS 503 vendu par la société BYK inférieure à 30, de préférence inférieure à 20, de façon encore plus préférée inférieure à 10.

La valeur de la brillance est obtenue par la moyenne de 3 mesures prises à trois points différents de la carte de contraste.

### Dureté de la composition

Un des avantages de la composition de l'invention est que sa texture est homogène et crémeuse et qu'elle le reste au cours du temps.

Cette propriété de stabilité de la texture au cours du temps se mesure grâce à une mesure de dureté.

La composition cosmétique de l'invention telle que définie ci-dessus peut encore être caractérisée en ce qu'elle présente une dureté inférieure à 300 g (grammes) et cela 1 jour après la fabrication et après un stockage de 1 jour, 15 jours, 1 mois et 2 mois dans une étuve à 45°C.

La dureté de la composition, qui est exprimée en grammes (g), est déterminée par la mesure de la force de compression mesurée à 20°C à l'aide d'un texturomètre vendu sous la dénomination « TA-XT Plus Microstable System » par la société Swantech. Le texturomètre est équipé d'un cylindre en inox d'un diamètre de 2 mm se déplaçant à la vitesse de mesure de 1mm/s et pénétrant dans la composition à une profondeur de 3 mm.

La valeur de la dureté est la force de compression mesurée, divisée par la surface du cylindre du texturomètre en contact avec la composition. Les échantillons sont coulés à chaud et à ras bord dans une boite de pétri ronde de taille 60 mm de rayon et 15 mm de hauteur. Les échantillons ainsi préparés sont conservés pendant 24 h à 48 h à 20°C avant d'effectuer la mesure.

La présente invention a également pour objet une composition de rouge à lèvres sous forme d'un bâton comprenant :
- De 8% à 20% en poids de charge matifiante par rapport au poids total de la composition
- De 5% à 8% en poids d'agent colorant par rapport au poids total de la composition
- De 10% à 30% et de préférence de 15% à 25% en poids d'agent structurant par rapport au poids total de la composition
- De 40% à 80%, et de préférence de 45% à 70% et de façon encore plus préférée de 50% à 60% d'huile par rapport au poids total de la composition.

### Procédé

La présente invention a également pour objet de fournir un procédé pour la préparation d'une composition de rouge à lèvres mate. Cet autre aspect de l'invention est caractérisé par le fait que l'on prépare une composition de rouge à lèvres mate par mélange d'au moins une charge particulière en tant qu'agent matifiant associée à au moins une huile et au moins un agent structurant. Une composition de rouge à lèvres obtenue selon le procédé de l'invention présente de manière avantageuse un aspect mat et une texture homogène et crémeuse.

La présente invention a également pour objet de fournir un procédé pour la préparation d'une composition de rouge à lèvres mate comprenant les étapes suivantes :
- (1) Broyer les pigments, si présents, préalablement dans une partie de l'huile ;
- (2) Faire fondre l'agent structurant et le reste de l'huile ;
- (3) Ajouter au mélange fondu obtenu en (2), la charge particulière comme agent matifiant, les éventuels agents colorants autres que les pigments et les éventuelles charges complémentaires ainsi que les éventuels pigments de l'étape (1) sous agitation continue ;
- (4) Ajouter optionnellement les actifs ou les parfums ; et
- (5) Couler à chaud la composition obtenue à l'étape (4) dans des moules, puis laisser refroidir jusqu'à solidification.

Les mesures de brillance et de dureté sont ensuite réalisées.

Les exemples suivants sont donnés afin d'illustrer l'invention. Ces exemples n'étant présentés qu'à titre d'illustration, l'invention ne peut en aucun cas être limitée à leur objet.

### Exemple I : PREPARATION DES ROUGES A LEVRES

On prépare des compositions de rouge à lèvres de formules suivantes (% en poids) :

**Table 1**

| | | | invention | | | | comparatif | | |
|---|---|---|---|---|---|---|---|---|---|
| | | | Ex 1 | Ex 2 | Ex3 | Ex4 | Ex 5 | Ex 6 | Ex 7 |
| fonction | Ingrédient | NOM COMMERCIAL | (%) | (%) | (%) | (%) | (%) | (%) | (%) |
| cire | Copolymère d'éthylène/propylène et cire synthétique | LIPWAX PZ80-20 | 11,10 | 11,10 | 11,10 | 11,10 | 11,10 | 11,10 | 11,10 |
| cire | Huile de ricin hydrogénée | CASTORWAX MP 80 | 3.46 | 3.46 | 3.46 | 3.46 | 3.46 | 3.46 | 3.46 |
| cire | Cire d'abeille naturelle | CERABEIL BLANCHE | 5,00 | 5,00 | 5,00 | 5,00 | 5,00 | 5,00 | 5,00 |
| huile | Isononyl isononanoate | DUB ININ | 17.54 | 17.54 | 17.54 | 17.54 | 17.54 | 17.54 | 17.54 |
| huile | Diisostéaryl malate | SALACOS 222 | 17.68 | 17.68 | 17.68 | 17.68 | 17.68 | 17.68 | 17.68 |
| huile | Dicaprilyl carbonale | CETIOL CC | 12.8 | 12.8 | 12.8 | 12.8 | 12.8 | 12.8 | 12.8 |
| épaississant | Dicaprilyl carbonale (8.56%) et stearalkonium hectorite (0.98%) et propylene carbonate (0.29%) | COSMEDIA GEL CC | 9.82 | 9.82 | 9.82 | 9.82 | 9.82 | 9.82 | 9.82 |
| actif | Tocopheryl acetate | DL-ALPHA-TOCOPHERYL ACETATE | 0,5 | 0,5 | 0,5 | 0,5 | 0,5 | 0,5 | 0,5 |
| Charge complémentaire | Magnésium aluminium silicate | NEUSILIN ULF2 | 1,00 | 1,00 | 1,00 | 1,00 | 1,00 | 1,00 | 1,00 |
| Agent colorant | Pigments | | 7,00 | 7,00 | 7,00 | 7,00 | 7,00 | 7,00 | 7,00 |
| parfum | Parfum | | 0,08 | 0,08 | 0,08 | 0,08 | 0,08 | 0,08 | 0,08 |
| charge de l'invention | Carbonate de magnésium basique | Basic magnésium carbonate de ICL Industrial products | 14,0 | - | - | - | - | - | - |
| charge de l'invention | Carbonate de magnésium basique traité triéthoxycaprylilsilane | Basic magnésium carbonate de ICL Industrial products traité triéthoxycaprylilsilane | - | 14,0 | - | - | - | - | - |
| charge de l'invention | Terre de diatomée | **IMERCARE^{®} 03D de IMERYS** | **-** | - | 14,0 | - | - | - | - |
| charge de l'invention | Cellulose microcristalline | **VIVAPUR ^{®}CS 4FM** | - | - | - | 14,0 | - | - | - |
| charge hors invention | Carbonate de magnésium plaquettaire PE | Carbonate de magnésium plaquettaire de SCORA | - | - | - | - | 14,0 | - | - |
| charge hors invention | Carbonate de magnésium tubulaire | MgTube^{®} de Nittetsu Mimming | - | - | - | - | - | 14,0 | - |
| charge hors invention | Methyl méthacrylate crosspolymer | TECHPOLYMER MBP-8 | - | - | - | - | - | - | 14,0 |
| Total | | Total composition : | 100 | 100 | 100 | 100 | 100 | 100 | 100 |

### Mode opératoire :

Les pigments sont broyés préalablement dans une partie des ingrédients huileux. Ensuite, on procède à la fusion des ingrédients structurants polymères ou cires et le reste des ingrédients huileux séparément et on ajoute les agents matifiants et charges complémentaires puis les pigments broyés au mélange précédent sous agitation continue. Enfin, on peut ajouter des actifs ou des parfums appropriés. On coule ensuite à chaud dans des moules. Puis on démoule après refroidissement.

### Exemple II: MESURES DE SURFACE SPECIFIQUE DES CHARGES MATIFIANTES

Les mesures de surface spécifique des charges matifiantes sont réalisées à l'aide d'un appareil de mesure BET tristar II. La méthode est basée sur l'adsorption des molécules d'azote à la surface et dans les pores de l'échantillon de poudre à basse température (température de liquéfaction de l'azote : 77°K). La surface spécifique est exprimée en m²/g.

**Table 2**

| exemple | Nom commercial | Nom INCI | fournisseur | taille particule | surface spécifique (m²/g) |
|---|---|---|---|---|---|
| Charge Ex 1 invention | Carbonate de magnésium basique | MAGNESIUM CARBONATE | ICL Industrial products | de 5 à 25µm | 4,1 |
| Charge Ex2 invention | Carbonate de magnésium basique traité triethoxycaprylyl-silane | MAGNESIUM CARBONATE | ICL Industrial products | 5 à 20µm | 4,7 |
| Charge Ex3 invention | **Imercare^{®} 03D** | TERRE DE DIATOMEE | IMERYS | 2 à 5 µm | 12 |
| Charge Ex4 invention | **VIVAPUR ^{®}CS 4FM** | CELLULOSE MICROCRYST ALLINE | RETTENM AIER | 0.9 0 9.4 µm | 5,7 |
| Charge Ex5 hors invention | MgC03 PE plaquettaire | MAGNESIUM CARBONATE | SCORA | 3 à 25µm | 30,4 |
| Charge Ex6 hors invention | MgTube^{®} | TUBULAR MAGNESIUM CARBONATE | Nittetsu Mimming | tube 20 à 60µm ovoide 1 à 15µm | 107,5 |
| Charge Ex7 hors invention | TECHPOLYMER MBP-8 | METHYL METHACRYLA TE CROSSPOLYM ER | Sekuisi Plastics co Ltd | 4,8 à 14 microns | 81,9 |

### Exemple III- Mesures de Brillance et de dureté

### Mesure de la brillance

La composition de rouge à lèvres de chaque exemple préparé dans l'exemple I est étalée sur une carte de contraste à fond blanc sur une épaisseur de 500 microns grâce à un étaleur automatique présente une valeur de brillance mesurée à 85° à l'aide d'un brillancemètre sous le nom de micro-TRI-gloss ou PICOGLOSS 503 vendu par la société BYK.

La valeur de la brillance est obtenue par la moyenne de 3 mesures prises à trois points différents de la carte de contraste et indiquée dans la table 3.

### Mesure de la dureté

La dureté de la composition, qui est exprimée en grammes (g), est déterminée par la mesure de la force de compression mesurée à 20°C à l'aide d'un texturomètre vendu sous la dénomination « TA-XT Plus Microstable System » par la société Swantech. Le texturomètre est équipé d'un cylindre en inox d'un diamètre de 2 mm se déplaçant à la vitesse de mesure de 1mm/s et pénétrant dans la composition à une profondeur de 3 mm.

La valeur de la dureté est la force de compression mesurée, divisée par la surface du cylindre du texturomètre en contact avec la composition.

Les échantillons des compositions de rouge à lèvres de chaque exemple préparé dans l'exemple I sont coulés à chaud et à ras bord dans une boite de pétri ronde de taille 60 mm de rayon et 15 mm de hauteur. Les échantillons ainsi préparés sont conservés pendant 24 h à 48 h à 20°C avant d'effectuer la mesure. Les résultats sont indiqués dans la table 3.

**Table 3 :**

| Nom commercial Charge matifiante | Exemple | Brillance à 85° au BYK | Dureté (g) J+1 | Dureté (g) J+15 | Dureté (g) J+1 mois | Dureté (g) J+ 2mois |
|---|---|---|---|---|---|---|
| basic magnésium carbonate | Exemple 1 invention | 7,3 +/-0,5 | 280 +/-4 | 267 +/-6 | 270 +/-3 | 257+/-4 |
| Magnésium carbonate traité triethoxyxaprylylsilane | Exemple 2 invention | 8,1 +/-0,4 | 230 +/-8 | 226 +/-10 | 227 +/-7 | 215+/-9 |
| Terre de diatoméee Imercare^{®} 03D | Exemple 3 invention | 0,4 +/-0,1 | 223+/-4 | 215+/- 12 | 217+/-13 | 214+/-6 |
| **VIVAPUR ^{®}CS 4FM** | Exemple 4 invention | 2,7+/-0,5 | 245+/-4 | 244+/-8 | 246+/-3 | 262+/-3 |
| MgC03 PE plaquettaire | Exemple 5 comparatif | 1,1 +/-0,2 | 400 +/-3 | 400+/-5 | 410+/-5 | 403+/-4 |
| MgTube^{®} | Exemple 6 comparatif | 1 +/-0,1 | 457 +/- 10 | 512 +/- 4 | 463 +/-9 | 484+/-5 |
| TECHPOLYMER MBP-8 | Exemple 7 comparatif | 2,5+/-0,6 | 322 +/-4 | 321 +/-5 | 336 +/-5 | 315+/-4 |

Les compositions de l'exemple 1 et de l'exemple 2 avec un carbonate de magnésium de surface spécifique répondant aux critères de l'invention présentent une texture homogène et crémeuse caractérisée par sa dureté qui reste stable à une valeur inférieure à 300 g (grammes) au cours du temps même après stockage 15 jours, 1 mois et 2 mois à 45°C.

Les compositions de l'exemple 3 avec une terre de diatomée de surface spécifique répondant aux critères de l'invention présentent une texture homogène et crémeuse caractérisée par sa dureté qui reste stable à une valeur inférieure à 300 g (grammes) au cours du temps même après stockage 15 jours, 1 mois et 2 mois à 45°C.

Les compositions de l'exemple 4 avec une cellulose microcristalline de surface spécifique répondant aux critères de l'invention présentent une texture homogène et crémeuse caractérisée par sa dureté qui reste stable à une valeur inférieure à 300 g (grammes) au cours du temps même après stockage 15 jours, 1 mois et 2 mois à 45°C.

Au contraire les compositions des exemples 5 et 6 comparatifs avec un carbonate de magnésium de surface spécifique ne répondant aux critères de l'invention présentent une texture beaucoup plus dure dès la fabrication, avec une valeur supérieure à 400 g (grammes) et qui augmente encore en dureté au cours du temps après stockage 15 jours, 1 mois et 2 mois à 45°C.

La composition de l'exemple 7 comparatif avec un methyl methacylique crosspolymer (Techpolymer MBP-8) de surface spécifique ne répondant aux critères de l'invention présente une texture beaucoup plus dure dès la fabrication avec une valeur supérieure à 300 g (grammes) et qui augmente encore en dureté au cours du temps après stockage 15 jours, 1 mois et 2 mois à 45°C.

## Revendications

1. Composition de rouge à lèvres d'aspect mat comprenant au moins un agent structurant, au moins une huile, au moins un agent colorant et au moins une charge particulière comme agent matifiant, **caractérisée en ce que** la charge particulière comme agent matifiant est choisie parmi les poudres de carbonate de magnésium, en particulier le carbonate de magnésium basique de formule (MgCO₃)₃.Mg(OH)₂.3H₂O ou de formule (MgCO₃)₄.Mg(OH)₂.5H₂O ou bien le carbonate de magnésium normal de formule (MgCO₃).xH₂O, parmi les terres de diatomées, et parmi la cellulose microcrystalline, ou un de leurs mélanges, les particules ayant une surface spécifique inférieure à 30 m²/g, de préférence inférieure à 20 m²/g, de façon encore plus préférée inférieure à 10m²/g et de façon encore plus avantageuse inférieure à 5 m2/g.

2. Composition de rouge à lèvres selon la revendication 1 comprenant au moins un agent structurant, au moins une huile, au moins un agent colorant et au moins une charge particulière comme agent matifiant, **caractérisée en ce que** la charge particulière comme agent matifiant est choisie parmi les poudres de carbonate de magnésium ayant une surface spécifique inférieure à 10m²/g et de façon encore plus avantageuse inférieure à 5 m²/g.

3. Composition de rouge à lèvres l'une quelconque des revendications 1 à 2, **caractérisée en ce que** la teneur totale en charge particulière comme agent matifiant dans la composition est comprise entre 8% et 20% et de préférence entre 10 % et 15 % en poids par rapport au poids total de la composition.

4. Composition de rouge à lèvres selon l'une quelconque des revendications 1 à 3, **caractérisée en ce que** l'huile est choisie parmi les huiles non volatiles siliconées ou hydrocarbonées ou un de leurs mélanges.

5. Composition de rouge à lèvres selon l'une quelconque des revendications 1 à 4, **caractérisée en ce que** la composition comprend moins de 5% d'huile volatile et de préférence est exempte d'huile volatile.

6. Composition de rouge à lèvres selon l'une quelconque des revendications 1 à 5, **caractérisée en ce que** l'huile non volatile est choisie parmi les (poly) esters et (poly) éthers synthétiques, en particulier les (poly) esters d'acides en C6-C20 et d'alcools en C6-C20, avantageusement ramifiés, tels que l'isononanoate d'isononyle; les di(alkyl en C6-C20) carbonates tels que le dicaprylyl carbonate commercialisé par BASF sous la dénomination Cetiol CC ; les acides gras ramifiés et/ou insaturés ; les polyesters de polyols, en particulier de (di) pentaérythritol, tels que le tétraoctanoate de pentaérythritol, les huiles de silicone telles que les polydiméthylsiloxanes linéaires de viscosité comprise entre 5×10-^{s}m²/s et 10-⁴m²/s ou un de leurs mélanges.

7. Composition de rouge à lèvres selon l'une quelconque des revendications 1 à 6, **caractérisée en ce que** l'huile non volatile est choisie parmi l'isononyl isononanoate, le dicaprylyl carbonate, le diisostéaryl malate, ou un de leurs mélanges.

8. Composition de rouge à lèvres selon l'une quelconque des revendications 1 à 7, **caractérisée en ce que** l'huile est présente dans une teneur comprise entre 40% et 80% en poids, de préférence entre 45% et 70% en poids, plus préférentiellement entre 50% et 60% en poids, par rapport au poids de la composition.

9. Composition de rouge à lèvres selon l'une quelconque des revendications 1 à 8, **caractérisée en ce que** l'agent structurant est une cire choisie parmi la cire d'abeille, la cire synthétique, la cire de polyéthylène ou leur mélange.

10. Composition de rouge à lèvres selon la revendication 9, **caractérisée** en ce la cire est présente dans la composition cosmétique de l'invention en une teneur comprise entre 10% et 30%, de préférence entre 15% et 25% en poids par rapport au poids total de la composition.

11. Composition de rouge à lèvres selon l'une quelconque des revendications 1 à 10, **caractérisée en ce que** la composition comprend en outre une charge complémentaire choisie parmi l'amidon, une argile ou leur mélange.

12. Composition de rouge à lèvres selon l'une quelconque des revendications 1 à 11, **caractérisée en ce que** l'agent colorant est choisi parmi les colorants hydrosolubles ou liposolubles, les pigments, les nacres, les laques ou un de leurs mélanges.

13. Composition de rouge à lèvres selon l'une quelconque des revendications 1 à 12, **caractérisée en ce que** la teneur totale en agent colorant dans la composition est comprise entre 5 % et 8% en poids par rapport au poids total de la composition.

14. Composition de rouge à lèvres selon l'une quelconque des revendications 1 à 13, **caractérisée en ce que** la composition de rouge à lèvres de l'invention étalée sur une carte de contraste présente une valeur de brillance mesurée à 85° à l'aide d'un brillancemètre sous le nom de micro-TRI-gloss ou PICOGLOSS 503 vendu par la société BYK inférieure à 30 de préférence inférieure à 20, de façon encore plus préférée inférieure à 10.

15. Composition de rouge à lèvres selon l'une quelconque des revendications 1 à 14, **caractérisée en ce qu'**elle présente une dureté mesurée à 20°C à l'aide d'un texturomètre vendu sous la dénomination « TA-XT Plus Microstable System » par la société Swantech inférieure à 300 g (grammes) et cela 1 jour après la fabrication et après un stockage de 1 jour, 15 jours, 1 mois et 2 mois dans une étuve à 45°C.

16. Composition de rouge à lèvres selon l'une quelconque des revendications 1 à 15 sous forme d'un bâton **caractérisé en ce qu'**il comprend:
- De 8% à 20% en poids de charge matifiante par rapport au poids total de la composition ;
- De 5% à 8% en poids d'agent colorant par rapport au poids total de la composition ;
- De 10% à 30% et de préférence de 15% à 25% en poids d'agent structurant par rapport au poids total de la composition ; et
- De 40% à 80%, et de préférence de 45% à 70% et de façon encore plus préférée de 50% à 60% d'huile par rapport au poids total de la composition.

17. Procédé de préparation de la composition de rouge à lèvres selon l'une quelconque des revendications 1 à 16, **caractérisé en ce que** l'on réalise les étapes suivantes :
- (1) Broyer les pigments, si présents, préalablement dans une partie de l'huile ;
- (2) Faire fondre l'agent structurant et le reste de l'huile ;
- (3) Ajouter au mélange fondu obtenu en (2), la charge particulière comme agent matifiant, les éventuels agents colorants autres que les pigments et les éventuelles charges complémentaires ainsi que les éventuels pigments de l'étape (1) sous agitation continue ;
- (4) Ajouter optionnellement les actifs ou les parfums ; et
- (5) Couler à chaud la composition obtenue à l'étape (4) dans des moules, puis laisser refroidir jusqu'à solidification.

18. Utilisation d'une charge particulière comme agent matifiant selon l'une quelconque des revendications 1 à 3 pour matifier sans durcir une composition selon l'une quelconque des revendications 1 à 16.

## Patentansprüche

1. Lippenstiftzusammensetzung mit mattem Aussehen, umfassend wenigstens ein Strukturierungsmittel, wenigstens ein Öl, wenigstens einen Farbstoff und wenigstens einen teilchenförmigen Füllstoff als mattierendes Mittel enthält, **dadurch gekennzeichnet, dass** der teilchenförmige Füllstoff als mattierendes Mittel unter den Magnesiumcarbonatpulvern gewählt ist, insbesondere aus basischem Magnesiumcarbonat der Formel (MgCO₃)₃.Mg(OH)₂.3H₂O oder der Formel (MgCO₃)₄.Mg(OH)₂.5H₂O oder normalem Magnesiumcarbonat der Formel (MgCO₃).xH₂O, aus Diatomeenerden und aus mikrokristalliner Cellulose, oder einer Mischung davon, wobei die Teilchen eine spezifische Oberfläche von weniger als 30 m²/g, bevorzugt weniger als 20 m²/g, noch stärker bevorzugt weniger als 10 m²/g und noch vorteilhafter weniger als 5 m²/g aufweisen.

2. Lippenstiftzusammensetzung nach Anspruch 1, umfassend wenigstens ein Strukturierungsmittel, wenigstens ein Öl, wenigstens einen Farbstoff und wenigstens einen teilchenförmigen Füllstoff als mattierendes Mittel, **dadurch gekennzeichnet, dass** der teilchenförmige Füllstoff als mattierendes Mittel aus Magnesiumcarbonatpulvern mit einer spezifischen Oberfläche von weniger als 10 m²/g und noch vorteilhafter, von weniger als 5 m²/g gewählt ist.

3. Lippenstiftzusammensetzung nach einem der Ansprüche 1 bis 2, **dadurch gekennzeichnet, dass** der Gesamtgehalt an teilchenförmigem Füllstoff als Mattierungsmittel in der Zusammensetzung zwischen 8 und 20 Gew.-% und bevorzugt zwischen 10 und 15 Gew.-%, bezogen auf das Gesamtgewicht der Zusammensetzung, beträgt.

4. Lippenstiftzusammensetzung nach einem der Ansprüche 1 bis 3, **dadurch gekennzeichnet, dass** das Öl aus nichtflüchtigen Silikon- oder Kohlenwasserstoffölen oder einer Mischung davon gewählt ist.

5. Lippenstiftzusammensetzung nach einem der Ansprüche 1 bis 4, **dadurch gekennzeichnet, dass** die Zusammensetzung weniger als 5 % flüchtiges Öl enthält und bevorzugt frei von flüchtigem Öl ist.

6. Lippenstiftzusammensetzung nach einem der Ansprüche 1 bis 5, **dadurch gekennzeichnet, dass** das nichtflüchtige Öl gewählt ist aus synthetischen (Poly-)Estern und (Poly-)Ethern, insbesondere (Poly-)Estern von vorteilhaft verzweigten C6-C20-Säuren und C6-C20-Alkoholen, wie Isononyl-Isononanoat; Di(C6-C20-alkyl)-carbonate wie das von der BASF unter dem Namen Cetiol CC vertriebene Dicaprylylcarbonat; verzweigten und/oder ungesättigten Fettsäuren; Polyester von Polyolen, inbesondere von (Di)pentaerythritol, wie Pentaerythritoltetraoctanoat, Silikonöle wie lineare Polydimethylsiloxane mit einer Viskosität zwischen 5×10⁻⁶m²/s und 10⁻⁴m²/s oder Mischungen davon.

7. Lippenstiftzusammensetzung nach einem der Ansprüche 1 bis 6, **dadurch gekennzeichnet, dass** das nichtflüchtige Öl aus Isononylisonononanoat, Dicaprylylcarbonat, Diisostearylmalat oder Mischungen davon gewählt ist.

8. Lippenstiftzusammensetzung nach einem der Ansprüche 1 bis 7, **dadurch gekennzeichnet, dass** das Öl mit einem Gehalt zwischen 40 und 80 Gew.-%, bevorzugt zwischen 45 und 70 Gew.-%, stärker bevorzugt zwischen 50 und 60 Gew.-%, bezogen auf das Gewicht der Zusammensetzung, vorliegt.

9. Lippenstiftzusammensetzung nach einem der Ansprüche 1 bis 8, **dadurch gekennzeichnet, dass** das Strukturierungsmittel ein Wachs ist, das aus Bienenwachs, synthetischem Wachs, Polyethylenwachs oder einer Mischung davon gewählt ist.

10. Lippenstiftzusammensetzung nach Anspruch 9, **dadurch gekennzeichnet, dass** das Wachs in der erfindungsgemäßen kosmetischen Zusammensetzung mit einem Gehalt zwischen 10 und 30 Gew.-%, bevorzugt zwischen 15 und 25 Gew.-%, bezogen auf das Gesamtgewicht der Zusammensetzung, enthalten ist.

11. Lippenstiftzusammensetzung nach einem der Ansprüche 1 bis 10, **dadurch gekennzeichnet, dass** die Zusammensetzung ferner einen zusätzlichen Füllstoff umfasst, der aus Stärke, einer Tonerde oder einer Mischung davon gewählt ist.

12. Lippenstiftzusammensetzung nach einem der Ansprüche 1 bis 11, **dadurch gekennzeichnet, dass** der Farbstoff aus wasser- oder fettlöslichen Farbstoffen, Pigmenten, Perlmutt, Lack oder Mischungen davon gewählt ist.

13. Lippenstiftzusammensetzung nach einem der Ansprüche 1 bis 12, **dadurch gekennzeichnet, dass** der Gesamtgehalt des Farbstoffs in der Zusammensetzung zwischen 5 und 8 Gew.-%, bezogen auf das Gesamtgewicht der Zusammensetzung, beträgt.

14. Lippenstiftzusammensetzung nach einem der Ansprüche 1 bis 13, **dadurch gekennzeichnet, dass** die erfindungsgemäße Lippenstiftzusammensetzung auf einer Kontrastkarte aufgebracht einen Glanzwert aufweist, der bei 85 ° mit einem Glanzmessgerät unter der Bezeichnung micro-TRI-gloss oder PICOGLOSS 503, das von der Firma BYK vertrieben wird, gemessen wird und unter 30, bevorzugt unter 20 und noch stärker bevorzugt unter 10 liegt.

15. Lippenstiftzusammensetzung nach einem der Ansprüche 1 bis 14, **dadurch gekennzeichnet, dass** sie eine Härte von weniger als 300 g (Gramm) aufweist, gemessen bei 20°C mit einem Texturometer, das unter der Bezeichnung "TA-XT Plus Microstable System" von der Firma Swantech vertrieben wird, und zwar 1 Tag nach der Herstellung und nach einer Lagerung von 1 Tag, 15 Tagen, 1 Monat und 2 Monaten in einem Wärmeschrank bei 45 °C.

16. Lippenstiftzusammensetzung nach einem der Ansprüche 1 bis 15 in Form eines Stiftes, **dadurch gekennzeichnet, dass** sie umfasst:
- 8 bis 20 Gew.-% mattierenden Füllstoff, bezogen auf das Gesamtgewicht der Zusammensetzung;
- 5 bis 8 Gew.-% Farbstoff, bezogen auf das Gesamtgewicht der Zusammensetzung;
- 10 bis 30 Gew.-% und bevorzugt 15 bis 25 Gew.-% Strukturierungsmittel, bezogen auf das Gesamtgewicht der Zusammensetzung; und
- 40 % bis 80 %, bevorzugt 45 % bis 70 % und noch stärker bevorzugt 50 % bis 60 % Öl, bezogen auf das Gesamtgewicht der Zusammensetzung.

17. Verfahren zur Herstellung der Lippenstiftzusammensetzung nach einem der Ansprüche 1 bis 16, **dadurch gekennzeichnet, dass** die folgenden Schritte durchgeführt werden:
- (1) Vermahlen der Pigmente, falls vorhanden, vorab in einem Teil des Öls ;
- (2) Schmelzen des Strukturierungsmittels und des Ölrests;
- (3) Zu der in (2) erhaltenen geschmolzenen Mischung werden unter ständigem Rühren der teilchenförmige Füllstoff als mattierendes Mittel, etwaige von den Pigmenten verschiedene Farbstoffe und etwaige zusätzliche Füllstoffe sowie etwaige Pigmente aus Schritt (1) hinzugefügt;
- (4) Optionale Zugabe von Wirk- oder Duftstoffen; und
- (5) Gießen der in Schritt (4) erhaltenen Zusammensetzung unter Wärmeeinwirkung in Formen und Abkühlenlassen bis zur Verfestigung.

18. Verwendung eines bestimmten Füllstoffs als Mattierungsmittel gemäß einem der Ansprüche 1 bis 3 zur Mattierung ohne Aushärtung einer Zusammensetzung nach einem der Ansprüche 1 bis 16.

## Claims

1. Matte lipstick composition comprising at least one structuring agent, at least one oil, at least one colouring agent, and at least one particular filler as a mattifying agent, **characterised in that** the particular filler serving as a mattifying agent is selected from magnesium carbonate powders, in particular basic magnesium carbonate of formula (MgCO₃)₃.Mg(OH)₂.3H₂O or of formula (MgCO₃)₄.Mg(OH)₂.5H₂O or indeed normal magnesium carbonate of formula (MgCO₃).xH₂O, from diatomaceous earths, and from microcrystalline cellulose, or one of the mixtures thereof, particles having a specific surface area of less than 30 m²/g, preferably less than 20 m²/g, more preferably still less than 10m²/g and even more advantageously less than 5 m2/g.

2. Lipstick composition according to claim 1, comprising at least one structuring agent, at least one oil, at least one colouring agent, and at least one particular filler as a mattifying agent, **characterised in that** the particular filler serving as a mattifying agent is selected from among magnesium carbonate powders having a specific surface area of less than 10m²/g and even more advantageously less than 5 m²/g.

3. Lipstick composition according to any one of claims 1 to 2, **characterised in that** the total content of particular filler serving as a mattifying agent in the composition is between 8% and 20% and preferably between 10% and 15% by weight with respect to the total weight of the composition.

4. Lipstick composition according to any one of claims 1 to 3, **characterised in that** the oil is chosen from among silicone or hydrocarbon non-volatile oils or one of the mixtures thereof.

5. Lipstick composition according to any one of claims 1 to 4, **characterised in that** the composition comprises less than 5% volatile oil and preferably is free from volatile oil.

6. Lipstick composition according to any one of claims 1 to 5, **characterised in that** the non-volatile oil is chosen from among synthetic (poly) esters and (poly) ethers, in particular (poly) esters of C6-C20 acids and C6-C20 alcohols, advantageously branched, such as isononyl isononanoate; di(C6-C20 alkyl) carbonates such as dicaprylyl carbonate marketed by BASF under the trade name Cetiol CC; branched and/or unsaturated fatty acids; polyesters of polyols, in particular of (di) pentaerythritol, such as pentaerythritol tetraoctanoate, silicone oils such as linear polydimethylsiloxanes of viscosity between 5×10⁻⁶m²/s and 10⁻⁴m²/s; or one of the mixtures thereof.

7. Lipstick composition according to any one of claims 1 à 6, **characterised in that** the non-volatile oil is chosen from among isononyl isononanoate, dicaprylyl carbonate, diisostearyl malate, or one of the mixtures thereof.

8. Lipstick composition according to any one of claims 1 to 7, **characterised in that** the oil is present at a content of between 40% and 80% by weight, preferably between 45% and 70% by weight, more preferentially between 50% and 60% by weight, with respect to the weight of the composition.

9. Lipstick composition according to any one of claims 1 to 8, **characterised in that** the structuring agent is a wax chosen from among beeswax, synthetic wax, polyethylene wax or the mixture thereof.

10. Lipstick composition according to claim 9, **characterised in that** the wax is present in the cosmetic composition according to the invention at a content of between 10% and 30%, preferably between 15% and 25%, the percentages being percentages by weight with respect to the total weight of the composition.

11. Lipstick composition according to any one of claims 1 to 10, **characterised in that** the composition further comprises an additional filler chosen from among starch, a clay or the mixture thereof.

12. Lipstick composition according to any one of claims 1 to 11, **characterised in that** the colouring agent is chosen from among water-soluble or liposoluble colorants, pigments, nacres, lacquers or one of the mixtures thereof.

13. Lipstick composition according to any one of claims 1 to 12, **characterised in that** the total content of colouring agent in the composition is between 5% and 8% by weight with respect to the total weight of the composition.

14. Lipstick composition according to any one of claims 1 to 13, **characterised in that** the lipstick composition according to the invention spread on a contrast chart has a gloss value measured at 85° using a glossmeter named micro-TRI-gloss or PICOGLOSS 503 sold by the company BYK of less than 30, preferably less than 20, more preferably less than 10.

15. Lipstick composition according to any one of claims 1 to 14, **characterised in that** it has a hardness measured at 20°C using a texture analyser sold under the trade name "TA-XT Plus Microstable System" by the company Swantech of less than 300 g (grams), 1 day after manufacture and after storage of 1 day, 15 days, 1 month and 2 months in an oven at 45°C.

16. Lipstick composition according to any one of claims 1 to 15 under the form of a stick **characterised in that** it comprises:
- From 8% to 20% by weight of mattifying filler with respect to the total weight of the composition;
- From 5% to 8% by weight of colouring agent with respect to the total weight of the composition;
- From 10% to 30% and preferably from 15% to 25% by weight of structuring agent with respect to the total weight of the composition; and
- From 40% to 80%, and preferably from 45% to 70% and more preferably from 50% to 60% of oil with respect to the total weight of the composition.

17. Method for preparing the lipstick composition according to any one of claims 1 to 16, **characterised in that** the following steps are carried out:
- (1) Grinding the pigments, if present, beforehand in a portion of the oil;
- (2) Melting the structuring agent and the rest of the oil;
- (3) Adding to the molten mixture obtained in (2), the particular filler as a mattifying agent, any colouring agents other than the pigments and any additional fillers as well as any pigments from step (1) under continuous stirring;
- (4) Optionally adding the active substances or fragrances; and
- (5) Pouring the hot composition obtained in step (4) into moulds, then allowing to cool until solidification.

18. Use of a particular filler as a mattifying agent according to any one of claims 1 to 3 in order to mattify without hardening a composition according to any one of claims 1 to 16.
